# EUROPEAN PATENT APPLICATION

(11) **EP 2 594 289 A2**
(43) Date of publication of application: **22.05.2013**
(21) Application number: 11807067.1
(22) Date of filing: 14.07.2011
(51) Int. Cl.: A61K 47/02, A61K 33/00, A61P 25/28, A61P 25/00

(54) **USE OF CARBON NANOTUBES FOR PREVENTING OR TREATING BRAIN DISEASE**

(30) Priority: 14.07.2010 KR 20100067721
(71) Applicant: Brainguard Co., Ltd., Seoul 153-803 (KR)
(72) Inventor: JEONG, Yoonhwa, Yongin-si Gyeonggi-do 448-160 (KR); LEE, Hyun Jung, Seoul 150-838 (KR); LEE, Do Yeon, Daejeon 305-720 (KR); NOH, Yoo Hun, Seoul 131-141 (KR); KIM, Do Hee, Seoul 120-762 (KR); KIM, Ok Hyeon, Seoul 134-856 (KR); PARK, Ji Ae, Seoul 156-030 (KR); LEE, Jiwon, Gwacheon-si Gyeonggi-do 427-040 (KR)
(74) Representative: Winkler, Andreas Fritz Ernst
(86) International application number: PCT/KR2011/005200
(87) International publication number: WO 2012/008782

(57) **Abstract**

The present invention relates to the use of carbon nanotubes for preventing or treating brain disease, and more particularly, to a composition for protecting cranial nerves, to a composition for preventing or treating neurodegenerative diseases or ischemic brain disease or to a composition for treating traumatic injuries to the central nervous system, containing carbon nanotubes as an active ingredient, to the use of carbon nanotubes for preparing a therapeutic agent for the abovementioned diseases and to a method for treating the abovementioned diseases. The composition of the present invention enables patients to recover from physical damage to the brain, exhibits superior efficacy for inhibiting the onset of Parkinson's disease and strokes in animal models for Parkinson's disease and strokes, and the cytotoxic effects of beta amyloid in beta amyloid toxicity tests. Therefore, the composition of the present invention can be effectively used in the preparation of medicine for protecting cranial nerves, therapeutic agents for preventing or treating brain disease, or therapeutic agents for treating traumatic injuries to the central nervous system.

## Description

### FIELD OF THE INVENTION

The present invention relates to the use of carbon nanotubes for preventing or treating brain disease, and more particularly, to a composition for protecting cranial nerves, to a composition for preventing or treating degenerative brain diseases or ischemic brain diseases or to a composition for treating traumatic central nervous system injuries, containing carbon nanotubes as an active ingredient, to the use of carbon nanotubes for preparing a therapeutic agent for the above-mentioned diseases and to a method for treating the above-mentioned diseases.

### DESCRIPTION OF THE RELATED ART

Degenerative brain diseases are major cause of nerve cell death in the brain, including a variety of diseases such as dementia, Parkinson's disease, stroke, and Huntington's disease. One of representative degenerative brain diseases, dementia is a brain disease representing a serious loss of global cognitive ability and it is caused by chronic or progressive brain disease to appear disorders in a number of high ranking cerebral functions such as memory, thinking, comprehension, calculation, learning and language judgment. However, the cause of dementia is not known accurately. There have been merely reported that several putative causes are damages of cholinergic neurons in cerebral base, reductions of the neurotransmitter, β-amyloid protein accumulation by inflammatory reactions and oxidative stress.

Parkinson's disease is the second most common degenerative neurological brain disease and occurs in 1 % of the population over the age of 65 and 5 % of the population over the age of 85. Characteristic clinical symptoms are persistent tremor, rigidity, stiffness, postural instability. Especially Alzheimer's disease and or Lou Gehrig's disease is accompanied in some patients. It suggests common pathologic mechanism between Parkinson's disease and Alzheimer's disease.

In the present, there is no treatment of Parkinson's disease. Only, dopamine precursor L-dopa or dopamine receptor catalysts are known as symptom improvement agent. However, these drugs cannot protect persistent dopaminergic neural loss to reduce effects of drugs rapidly within 5-6 years, whereby side effects such as dyskinesias are increased and these lead to increase L-dopa problem known as "on-off" phenomenon in late stage of Parkinson's disease. After 10 to 15 years of the onset of Parkinson's disease, the symptoms become progressively worse with time and ultimately result in death.

In a modern society, even though increases of degenerative brain diseases (*e.g*. Alzheimer's disease, Parkinson's disease and senile dementia) by rapid increases of elderly population has become a serious social problem, effective drugs or treatments for preventing or treating these diseases have not been developed. Therefore, there is a need for treatment agents for delaying or protecting the process of degenerative brain diseases such as Alzheimer's disease and Parkinson's disease.

Ischemic brain diseases and hemorrhagic cerebrovascular diseases are representative cerebrovascular diseases. Hemorrhagic cerebrovascular diseases are occurred mainly by traffic accidents and ischemic brain diseases are occurred mainly in elderly people. Ischemic brain diseases are subdivided into thrombosis, embolism, transient ischemic attack and lacune. Ischemic brain diseases are being happen pathologic abnormality by thrombus and embolus in blood vessel in which supply bloodstream to brain.

Where transient cerebral ischemia is induced in brain, ATP reduction and edema in nerve cells are occurred by blocking supplements of oxygen and glucose in brain tissue to induce widespread brain damages such as stroke, cerebral infarction, cerebral circulation disorder and coma, eventually.

Drugs for ischemic brain diseases are platelet aggregation inhibitors such as ticlopidine, cilostazole and prostacycline, and anti-thrombin agents. These drugs have side effects, for example, headaches, palpitations and huge stress on liver, thereby they are limited on use.

Treatments for stroke are acute treatments and prevention treatments. There is known that acute treatments are effective for acute stroke within 3 hours after stroke and if not, there are no effects. In other words, it is well known that once cerebrovascular diseases occur, paucity of movement, sexual dysfunction and failure of memory are induced by irreversible neuronal damage. When the characteristics of the ischemic brain diseases consider, it tends to emphasize preventions rather than treatment. Need for prevention of cerebrovascular disease by persistent injecting substances in which can protect cranial nerve cells has been recognized newly. In addition, there is a need for developments of drugs which are safe and effective for protecting neuronal damage cause by ischemic stroke.

Traumatic brain injury (TBI) or traumatic spinal cord injury (TSCI) occur as a result of damage on the central nervous system by accidents, sudden movements or physical shocks. As they are often referred to closed head injury (CHI), early treatment is very important in order to prevent additional damage after the initial treatment is very important in order to prevent additional damage after the initial injury. Main forms of traumatic nerve injury are diffuse axonal injury, contusion, anoxic, hemorrhagic and perfusion-repferfusion. In most of brain injury cases, the dominant mechanism is diffuse axonal injury. Axonal injury is common injury in all traumatic nerve injury without regard to the relative seriousness. Diffuse axonal injury in human, progressive change is occurred by axonal distortion to result loss of connections between nerve cells.

500,000 of new cases for traumatic brain injury are determined each year in the United States. There is estimated that 0.2 % of the cases is required hospitalization. In the case of degenerative brain disease of Korea, senile dementia occurs in 10 % of the population over the age of 65 and Parkinson's disease occurs in approximately 1 % of the population over the age of 65. In addition, ischemic stroke is one of main death causes as the second highest following cancer. In every year, 74 people per 100,000 population die by the stroke according to 2001 data from the National Statistical Office. The brain diseases are adult diseases that incidence are rapidly increased in the elderly people over the age of 50. They are not only serious social financial burden but also causes of qualitative degradation of life in elderly people.

Moreover, entire changes of modern society (*e.g*. increases of transportation, extension of the average life expectancy and westernized eating habits) result in persistent increase of traumatic nerve injury, degenerative brain disease and ischemic brain disease such that there is a need for developments of therapeutic agents for preventing and treating these injuries and diseases

Carbon nanotubes (CNTs) are allotropes of carbon with a cylindrical nanostructure having a diameter measuring on the nanometer scale (nm= one-billionth of a meter). CNTs are known as new ideal material which has unusual properties; excellent mechanical properties, electrical selectivity, remarkable field emission and hydrogen storage medium of high efficiency, and no defects compared to conventional material in existence.

CNTs have excellent dynamic robustness and chemical stability and act either as semiconductor or as conductor. Their diameters are nanometer scale and their lengths are relatively very long. Owing to these properties, CNTs are valuable as material for flat panel display device, transistors, energy storage device. In addition, of CNTs are expected to be very good applicability as nano-size electronic element.

Recently, CNTs have been attracted tremendous interest by high electrical conductivity, hollow geometry, adsorptive ability, good mechanical strength and excellent biocompatibility. There is reported that CNTs are applied in the field of biotechnology, for example, glucose sensor, detection of protein and detection of specific DNA sequences (Anal. Bioanal. Chem., 375:103-5, 2003; Proc. Natl. Acad. Sci. USA, 100 (9) :4984-9, 2003; Anal. Bioanal. Chem., 375:287-93, 2003).

However, use of CNTs in the field of biotechnology is limited to materials for diagnostics and test and there is no application for protecting and treating cranial nerve and brain disease using CNTs as an active ingredient. Especially, there has been no effectiveness of CNTs for protecting cranial nerve or effectiveness of CNTs for preventing or treating degenerative brain disease, ischemic brain diseases and traumatic central nervous system damage.

Throughout this application, several patents and publications are referenced and citations are provided in parentheses. The disclosure of these patents and publications is incorporated into this application in order to more fully describe this invention and the state of the art to which this invention pertains.

### DETAILED DESCRIPTION OF THIS INVETNION

### Technical Purposes of This Invention

The present inventors have made intensive studies to develop novel approach for protecting central nerves from various causes such as trauma and diseases. As a result, the present inventors have found out that carbon nanotubes are significantly effective in protection of central nerves.

Accordingly, it is an object of the present invention to provide a composition for preventing or treating a brain disease, comprising a carbon nanotube as an active ingredient.

It is another object of the present invention to provide a composition for treating traumatic central nervous system injuries, comprising a carbon nanotube as an active ingredient.

It is still another object of the present invention to provide a composition for protecting cranial nerves, comprising a carbon nanotube as an active ingredient.

It is further object of the present invention to provide a use of a carbon nanotube for manufacturing a therapeutic agent for preventing or treating a brain disease.

It is still further object of the present invention to provide a use of a carbon nanotube for manufacturing a therapeutic agent for preventing or treating traumatic central nervous system injuries.

It is another object of the present invention to provide a use of a carbon nanotube for manufacturing a medicament for protecting cranial nerves.

It is still another object of the present invention to provide a method for preventing or treating a brain disease, comprising administering to a subject in need thereof a therapeutically effective amount of a carbon nanotube.

It is further object of the present invention to provide a method for preventing or treating a traumatic central nervous system injury, comprising administering to a subject in need thereof a therapeutically effective amount of a carbon nanotube.

It is still further object of the present invention to provide a method for protecting cranial nerves, comprising administering to a subject in need thereof a therapeutically effective amount of a carbon nanotube.

### Technical Solutions of This Invention

In one aspect of the present invention, there is provided a composition for preventing or treating a brain disease, comprising a carbon nanotube as an active ingredient.

In another aspect of the present invention, there is provided a composition for treating traumatic central nervous system injuries, comprising a carbon nanotube as an active ingredient.

In still another aspect of the present invention, there is provided a composition for protecting cranial nerves, comprising a carbon nanotube as an active ingredient.

In further aspect of the present invention, there is provided a use of a carbon nanotube for manufacturing a therapeutic agent for preventing or treating a brain disease.

In still further aspect of the present invention, there is provided a use of a carbon nanotube for manufacturing a therapeutic agent for preventing or treating traumatic central nervous system injuries.

In another aspect of the present invention, there is provided a use of a carbon nanotube for manufacturing a medicament for protecting cranial nerves.

In still another aspect of the present invention, there is provided a method for preventing or treating a brain disease, comprising administering to a subject in need thereof a therapeutically effective amount of a carbon nanotube.

In further aspect of the present invention, there is provided a method for preventing or treating a traumatic central nervous system injury, comprising administering to a subject in need thereof a therapeutically effective amount of a carbon nanotube.

In still further aspect of the present invention, there is provided a method for protecting cranial nerves, comprising administering to a subject in need thereof a therapeutically effective amount of a carbon nanotube.

The present invention provides a composition for preventing or treating a brain disease, comprising a carbon nanotube as an active ingredient.

The present invention provides a composition for treating traumatic central nervous system injuries, comprising a carbon nanotube as an active ingredient.

The present invention provides a composition for protecting cranial nerves, comprising a carbon nanotube as an active ingredient.

The present composition contains a carbon nanotube as an active ingredient. As used herein, the carbon nanotube is allotrope of carbon with a cylindrical nanostructure having a diameter measuring on the nanometer scale. The present carbon nanotube may exist as multilayered shell, multi-wall nanotube or single-wall nanotube. Preferably, the present carbon nanotube is single-wall nanotube. More preferably, the present carbon nanotube is single-wall nanotube having 4-5 nm of diameter and 0.5-1.5 µm of average length.

Preferably, the present carbon nanotube is functionalized. The functionalized carbon nanotube allows for the decrease in aggregation between CNT molecules. The carbon nanotube is functionalized with one or more functional groups selected from the group consisting of carboxyl group, thiol group, amide group, poly(ethylene glycol) group (PEG group), octadecylamine group (ODA group) and polyaminobenzene sulfonic acid group (PABS group). The kind of carbon nanotube prepared by functional group in this invention may be the carbon nanotube represented by the following <General formula 1>, but not limited to.

### <General formula 1>

| | | |
|---|---|---|
| 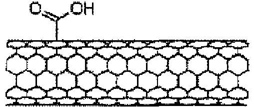 | 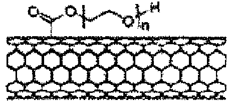 | 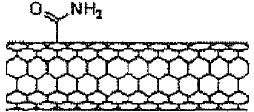 |
| <carboxylic CNT> | <PEG-CNT> | <amide-CNT> |
| 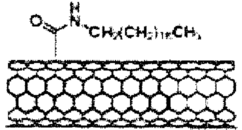 | 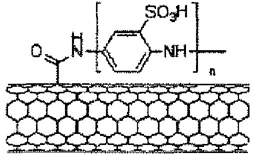 | |
| <ODA-CNT> | <PABS-CNT> | |

The carbon number of the present carbon nanotube is not limited, preferably C₂₀-C₁₅₀. The present carbon nanotube may be prepared in accordance with various processes known in the art. The present carbon nanotube may exist as various forms, preferably suspension.

The present composition containing a carbon nanotube as an active ingredient has an excellent efficacy for preventing or treating a brain disease of animals including human.

A brain disease in the present invention refers to all kinds of the diseases which are occurred in brain of animals including human, preferably a degenerative brain disease or an ischemic brain disease. The present composition containing a carbon nanotube as an active ingredient has an excellent efficacy for preventing or treating a degenerative brain disease or an ischemic brain disease. The degenerative brain disease in the present invention is selected from the group consisting of Alzheimer's disease, Parkinson's disease, dementia, progressive supranuclear palsy, multiple system strophy, Olivopontocerebellar atrophy (OPCA), Shy-Drager syndrome, Striatonigral degeneration, Huntington's disease, amyotrophic lateral sclerosis (ALS), essential tremor), cortico-basal ganlionic degeneration, Diffuse Lewy body disease, Parkinson-ALS-dementia complex of Guam and Pick's disease.

Ischemic brain diseases are being happen various pathologic abnormalities in blood vessel in which supply bloodstream to brain.

The ischemic brain disease in the present invention is selected from the group consisting of thrombosis, embolism, transient ischemic attack, lacune, stroke, cerebral hemorrhage, cerebral infarction, head trauma, cerebral circulation disorder and coma,

In addition, the present composition containing a carbon nanotube as an active ingredient has an excellent efficacy for treating traumatic central nervous system injuries.

The traumatic central nervous system injuries in the present invention refer to physical damage in central nervous system including brain and spinal cord, preferably traumatic brain injury (TBI) or traumatic spinal cord injury (TSCI).

These efficacies of the present carbon nanotube are first investigated by the present inventor.

The efficacies of the present invention as described above have been proven in embodiments.

In one embodiment of the present invention, the traumatic brain injury animal model is prepared with ICR mice. The present composition containing the carbon nanotube is injected into the damaged area of the mouse. After 28 days, passive avoidance test is subject to analyze the acquiring and maintenance of learning and memory of the mouse having traumatic brain injury. In addition, the mouse is sacrificed to observe brain tissue of the damaged area.

As a result, it is determined that while most of the group injected with PBS did not recognize the shock, most of the group injected with the carbon nanotubes of the present invention effectively recovered learning memory ability. In addition, it is determined that while traumatic brain injury in the mouse injected with PBS is progressed to make hole in brain tissue, traumatic brain injury in the mouse injected with the carbon nanotubes of the present invention is regenerated to heal damaged area in brain tissue (see Example 2).

Therefore, it could be understood that the present composition has excellent efficacies for protecting cranial nerves and treating traumatic central nervous system injuries.

In another embodiment of the present invention, an efficacy of the present composition for Parkinson's disease is verified. First, the carbon nanotubes composition is treated to neuroblast cell line SH-SY5Y, followed by treatment of 6-hydroxydopamine to induce neuronal cell damage and measured level of neuronal cell damage. In addition, the Parkinson's disease animal model is prepared with ICR mice. The present composition containing the carbon nanotube is injected into the mouse. The mouse is injected with 6-hydroxydopamine to induce neuronal cell damage. After 2 weeks, rotation test is performed with apomorphine.

As a result, it is determined that while the mitochondrial membrane potential at 6hrs after 6-hydroxydopamine treatment in control group decreased remarkably by 40 % of normal level, the mitochondrial membrane potential in the carbon nanotubes of the present invention maintained with no more less 60 %. In addition, it is determined that the asymmetric rotation numbers in the group injected with the present composition decreased remarkably as compared to the group injected with PBS (see Example 3).

Therefore, it could be understood that the present composition has excellent efficacies for protecting cranial nerves, and for preventing and treating degenerative brain disease.

In still another embodiment of the present invention, an efficacy of the present composition for stroke is verified. First, the present composition is treated to the rat. After 7 days, an ischemic stroke model is prepared by transient middle cerebral artery occlusion (MCAO) with the rat. Following this, Rotarod test is subjected to evaluate brain function damage and sensomobility of forelimb and hindlimb responding to stimuli for senses of vision and position is executed through Limb placing test. In addition, occurrence of stroke is verified through histological observation.

As a result, latency time of the group injected with PBS is remarkably decreased in Rotarod test after MCAO induction and 7 days after MCAO induction, the latency time is not recovered remarkably. In contrast, latency time of the group injected with the present composition is represented more than 150 sec after MCAO induction. In addition, 7 days after MCAO induction, latency time of that is recovered to nearly normal state.

Limb placing test can evaluate damage on sensomobility. It is determined that the group injected with the present composition represents remarkably lower neuronal damage on 1 day after MCAO induction as compared to PBS group and is still maintained on 7 days after induction.

To verify the effect of the carbon nanotube for inflammatory responses of CA1 and CA3, immunohistochemistry is carried out using GFAP (glial fibrillary acidic protein) which is a marker for glial activation. As a result, it is determined that the star-shaped size and the number of astrocytes in the group injected with PBS were increased and activated. Such results indicate that inflammatory responses were increased. In contrast, it is determined that glial activation level in the group injected with the carbon nanotubes at 7 days before induction of MCAO is maintained in the same level of sham group (see Example 4). Such result indicates that the present carbon nanotubes enable the nerve cells and brain tissue to endure against MCAO, and it has excellent efficacies for protecting cranial nerves, and for treating an ischemic brain disease.

In further embodiment of the present invention, the present composition effects for Alzheimer's disease were verified. Human neuroblast cell line SH-SY5Y is treated with the present composition and treated with amyloid beta peptide. After 36 hrs, the neuronal cells were measured their viabilities mitochondrial membrane potential.

As a result, it is determined that while the cell death in the group non-treated carbon nanotubes after treatment of amyloid beta is represented by approximately 50 %, the cell viability in the group injected with the carbon nanotubes before 1 hr is increased by approximately 70%. In addition, it is determined that the present carbon nanotubes may inhibit damage of mitochondrial membrane potential caused by amyloid beta and protect function of mitochondrial membrane potential. Such result indicates that the present carbon nanotube may provide effects for protecting cranial nerves, and for preventing and treating degenerative brain disease in cellular level as well as in tissue level (see Example 5).

The pharmaceutical composition of the present invention may further include pharmaceutically acceptable carrier, excipient or diluent. An amount of the carbon nanotube in the composition may be preferably present in the ranges from 0.001 to 50 wt%. Where the amount of the carbon nanotube in the composition is below 0.001 wt%, the higher frequency of the administration is likely to be required for effective efficacy; in the case of exceeding 50 wt%, the increase in its efficacy may be failed as increasing dosage.

The terms used herein "pharmaceutically acceptable" refers to a non-toxic and physiological-tolerable composition that does not induce gastrointestinal troubles as well as allergic responses or their similar responses such as dizziness, and exhibits compatibility with active ingredients when administered to a human subject.

In this invention, the pharmaceutically acceptable carrier may further include carrier for oral administration or parenteral administration. The carrier for oral administration may include lactose, starch, cellulose derivatives, magnesium stearate, stearic acid. In addition, variety of drug delivery substance used in peptide formulation for oral administration may included. The carrier for parenteral administration may include water, suitable oil, saline, aqueous glucose and glycol, and further include stabilizers and preservatives. Appropriate stabilizers are antioxidants such as sodium hydrogen sulfite, sodium sulfite or ascorbic acid. Appropriate preservatives are benzalkonium chloride, methyl-or propylparaben and chlorobutanol. The pharmaceutical composition according to the present invention may further include a lubricant, a humectant, a sweetener, a flavoring agent, an emulsifier and a suspending agent. Details of suitable pharmaceutically acceptable carriers and formulations can be found in Remington's Pharmaceutical Sciences (19th ed., 1995), which is incorporated herein by reference.

The pharmaceutically composition containing the present carbon nanotube as an active ingredient may separately include a pharmaceutically effective amount of a carbon nanotube, or further include a pharmaceutically acceptable carrier. The term used herein "pharmaceutically effective amount" refers to an amount suitable to show and accomplish efficacies and activities of the present invention. The degenerative brain disease is Alzheimer's disease, Parkinson's disease, dementia, progressive supranuclear palsy, multiple system strophy, Olivopontocerebellar atrophy (OPCA), Shy-Drager syndrome, Striatonigral degeneration, Huntington's disease, amyotrophic lateral sclerosis (ALS), essential tremor), cortico-basal ganlionic degeneration, Diffuse Lewy body disease, Parkinson-ALS-dementia complex of Guam or Pick's disease. The ischemic brain disease is thrombosis, embolism, transient ischemic attack, lacune, stroke, cerebral hemorrhage, cerebral infarction, head trauma, cerebral circulation disorder or coma. The traumatic central nervous system injuries are traumatic brain injury (TBI) or traumatic spinal cord injury (TSCI).

The composition of this invention may be administered by any administation methods to mammals including humans. For example, the composition of this invention may be administered orally or parenterally. The parenteral administration may be administered by intravenously, intramuscularly, intraarterially, intramedullary, intradurally, intracardially, transdermal, subcutaneously, intraperitoneally, nasally, intestinally, topically, sublingually or rectally, but not limited to.

The pharmaceutical composition of the present invention may be formulated for oral administration or parenteral administration in accordance with administration routes as mentioned above.

When the composition of the present disclosure is prepared as oral administration, the composition of the present may be formulated as powders, granules, tablets, pellets, sugarcoated pills, capsules, liquid medicines, gels, syrups, slurries and suspensions by methods known in the art. When the composition of the present disclosure is prepared as parenteral administration, the composition of the present invention may be formulated in the form of injections, creams, lotions, topical ointments, oils, moisturizers, gels, aerosol and nasal inhalants by methods known in the art. For example, these parenteral administrations may be prepared by mixing the composition with a suitable excipient in the form of solution in oily or aqueous medium, suspension or emulsion. Examples of suitable excipients may include carbohydrates (*e.g.* lactose, amylase, dextrose, sucrose, sorbitol, mannitol, xylitol, erythritol and maltitol), starches (*e.g.* corn starch, wheat starch, rice starch, potato starch), celluloses (cellulose, methyl cellulose, sodium carboxymethyl cellulose, hydroxypropyl methyl-cellulose) and fillers (gelatin, polyvinylpyrrolidone). In addition, in some cases, disintegrants such as cross-linked polyvinylpyrrolidone, agar, alginic acid or sodium alginate may be added. Furthermore, the pharmaceutical composition of the present invention may further include anticoagulants, lubricants, wetting agents, flavoring agents, emulsifiers and preservatives.

Details of suitable pharmaceutically acceptable carriers and formulations can be found in Remington's Pharmaceutical Sciences (19th ed., 1995), which is incorporated herein by reference.

Total therapeutically effective amount of the present composition may be administered to patients with single dose or multiple dose of fractionated treatment protocol. A suitable content of the active ingredient of the pharmaceutical composition in the present invention may vary depending on the extent of the diseases. Preferably, the pharmaceutical composition of the present invention is administered with a daily dose of 0.001 µg/kg-1,000 mg/kg (body weight of patient), most preferably 0.01 µg/kg-100 mg/kg. However, an appropriate administration dosage of the pharmaceutical composition of the present disclosure may be determined variously depending on such factors as an administration route, an administration time, an age, a body weight and a gender of a patient, a pathological condition, a severity of diseases, a diet and an excretion rate. Therefore, physicians of ordinary skill in the art may determine an effective amount of the pharmaceutical composition for desired treatment. The pharmaceutical composition according to the present invention is not specifically limited to the formulation, the administration route and the administration method as long as it serves the effects of the present invention.

The present invention provides a use of a carbon nanotube for manufacturing a therapeutic agent for preventing or treating a brain disease.

In addition, the present invention provides a use of a carbon nanotube for manufacturing a therapeutic agent for preventing or treating traumatic central nervous system injuries.

In addition, the present invention provides a use of a carbon nanotube for manufacturing a medicament for protecting cranial nerves.

In addition, the present invention provides a method for preventing or treating a brain disease, comprising administering to a subject in need thereof a therapeutically effective amount of a carbon nanotube.

In addition, the present invention provides a method for preventing or treating a traumatic central nervous system injury, comprising administering to a subject in need thereof a therapeutically effective amount of a carbon nanotube.

In addition, the present invention provides a method for protecting cranial nerves, comprising administering to a subject in need thereof a therapeutically effective amount of a carbon nanotube.

The present carbon nanotube may be administrated through various routes such as oral, transdermal, subcutaneous, intravenous or intramuscular with a therapeutically effective amount. The term used herein "therapeutically effective amount" refers to an amount suitable to show and accomplish effects for treating a brain disease or traumatic central nervous system injuries, or for protecting cranial nerves.

The term used herein "subject" refers to animals, preferably mammals, particularly humans, a cell derived from, a tissue derived from, an organ derived from animal or a patient in need treatment.

The present carbon nanotube may be administrated separately or with various forms as mentioned above, preferably to obtain desired effects, *i.e,* to accomplish effects for treating a brain disease or traumatic central nervous system injuries, or for protecting cranial nerves.

### Effects of This Invention

The features and advantages of the present invention will be summarized as follows:

The present composition provides a composition for preventing or treating a brain disease, a composition for treating traumatic central nervous system injuries, a composition for protecting cranial nerves, use and method of a carbon nanotube for manufacturing a therapeutic agent for the diseases as mentioned above.

The composition of the present invention enables patients to recover from physical damage to the brain, exhibits superior efficacy for inhibiting the onset of Parkinson's disease and strokes in animal models for Parkinson's disease and strokes, and the cytotoxic effects of beta amyloid in beta amyloid toxicity tests. Therefore, the composition of the present invention can be effectively used in the preparation of medicine for protecting cranial nerves, therapeutic agents for preventing or treating brain disease, or therapeutic agents for treating traumatic injuries to the central nervous system.

### Brief Description of the Drawings

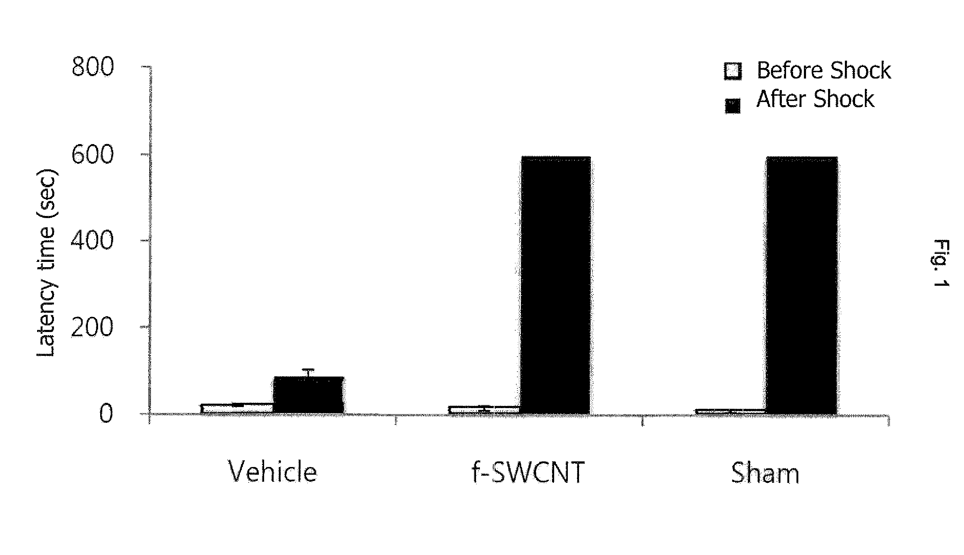

Fig. 1 represents graph of result that the present composition containing the carbon nanotube as an active ingredient is administrated to the traumatic brain injury animal model and the passive avoidance test is subject (Vehicle: PBS-treated group (control), f-SWCNT: the present composition-treated group, sham: substance-non treated and non operated group).

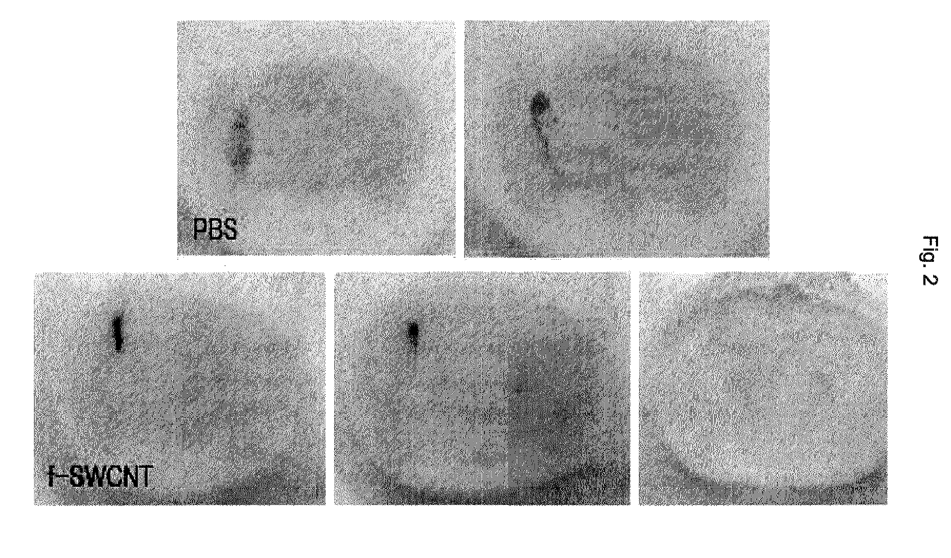

Fig. 2 represents images of brain section that the present composition containing the carbon nanotube as an active ingredient is administrated to the traumatic brain injury animal model and the brain tissue recovery is verified (PBS: PBS-treated group (control), f-SWCNT: the present composition-treated group).

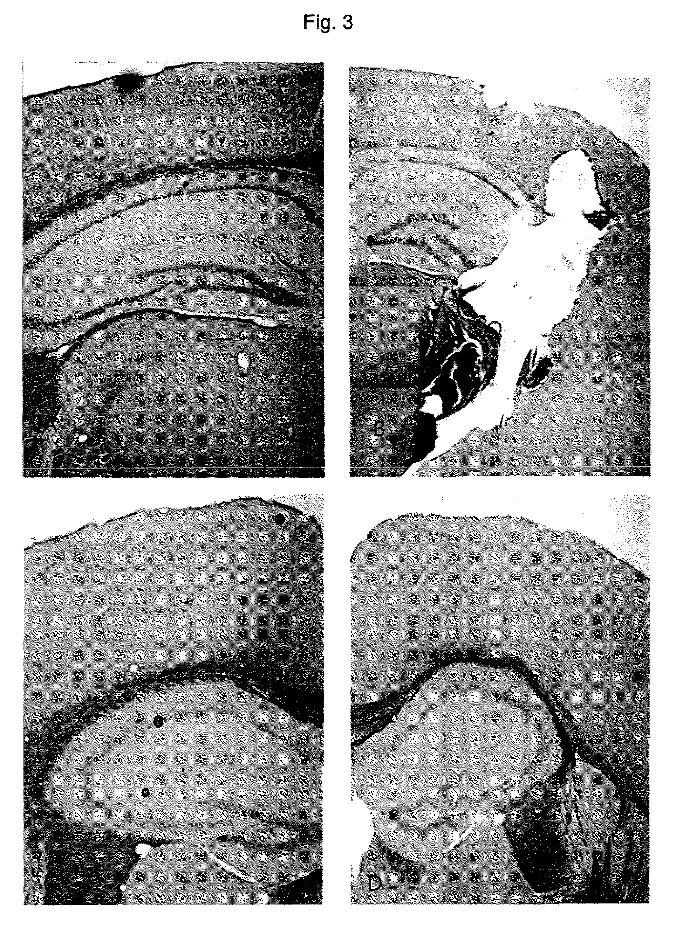

Fig. 3 represents images of brain section that the present composition containing the carbon nanotube as an active ingredient is administrated to the traumatic brain injury animal model and the brain tissue recovery is verified. Fig. 3a is a contralateral side for damaged area of PBS-treated group, Fig. 3b is an ipsilateral side for damaged area of PBS-treated group, Fig. 3c is a contralateral side for damaged area of group the present composition-treated group and Fig. 3d is an ipsilateral side for damaged area of the present composition-treated group.

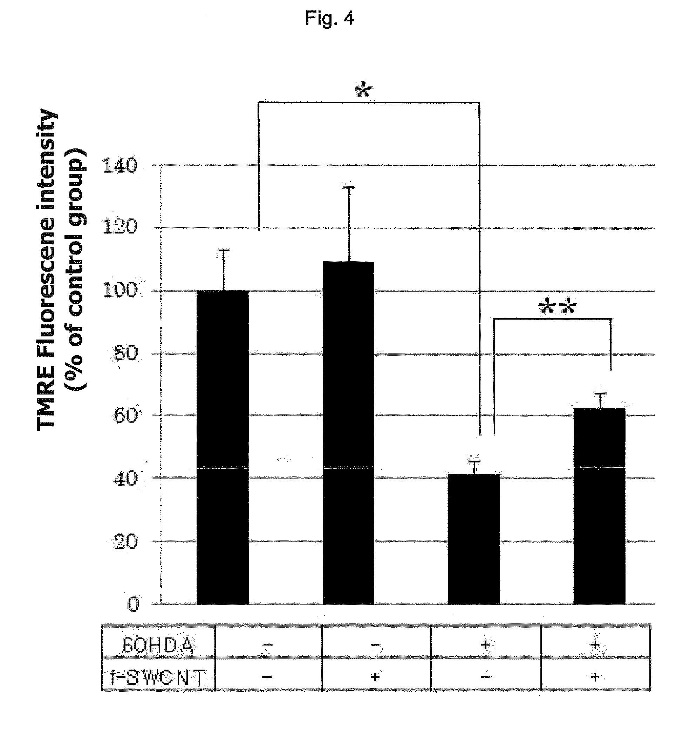

Fig. 4 represents graph of result that the present composition containing the carbon nanotube as an active ingredient is administrated to the Parkinson's disease model induced by 6-hydroxydopamine and inhibited the neuronal cell damage to verify by measuring mitochondrial membrane potential (6OHDA: 6-hydroxydopamine, f-SWCNT: the present composition-treated group, Y axis: TMRE (tetramethyl rhodamine ethyl ester) Fluorescene intensity (% of control group), *: P<0.01 change of 6OHDA-treated group as compared to control group, **: P<0.01 change of SWCNT group as compared to 6OHDA-treated group).

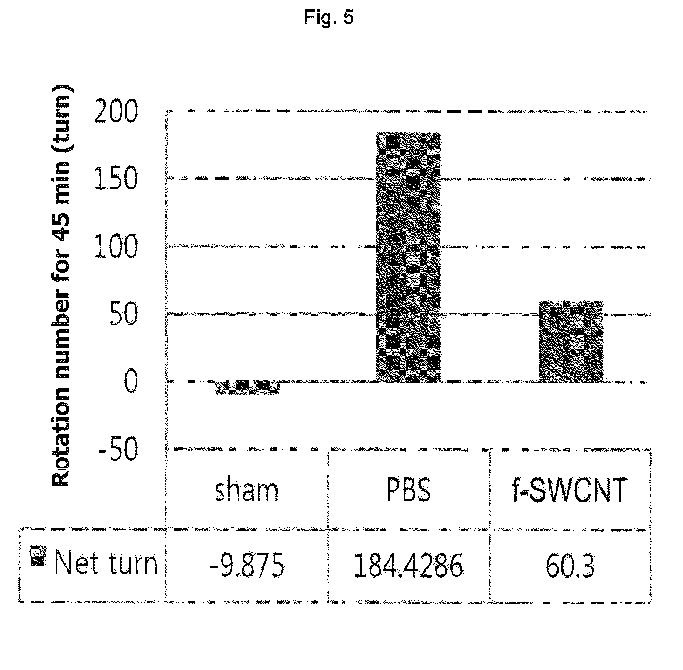

Fig. 5 represents graph of result that the present composition containing the carbon nanotube as an active ingredient is administrated to the Parkinson's disease model induced by 6-hydroxydopamine and inhibited the neuronal cell damage to verify by asymmetric rotation test (PBS: PBS-treated group (control), f-SWCNT: the present composition-treated group, sham: 6OHDA-non injected group, Y axis: Rotation number with one-direction for 45 min (unit: turn)).

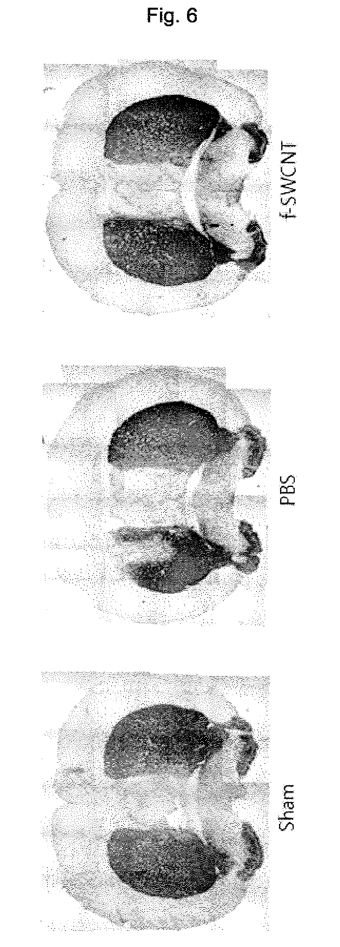

Fig. 6 represents images of result that the present composition containing the carbon nanotube as an active ingredient is administrated to the Parkinson's disease model induced by 6-hydroxydopamine and observed to verify inhibitory for loss of dopaminergic neuronal cell (PBS: PBS-treated group (control), f-SWCNT: the present composition-treated group, sham: 6OHDA-non injected group).

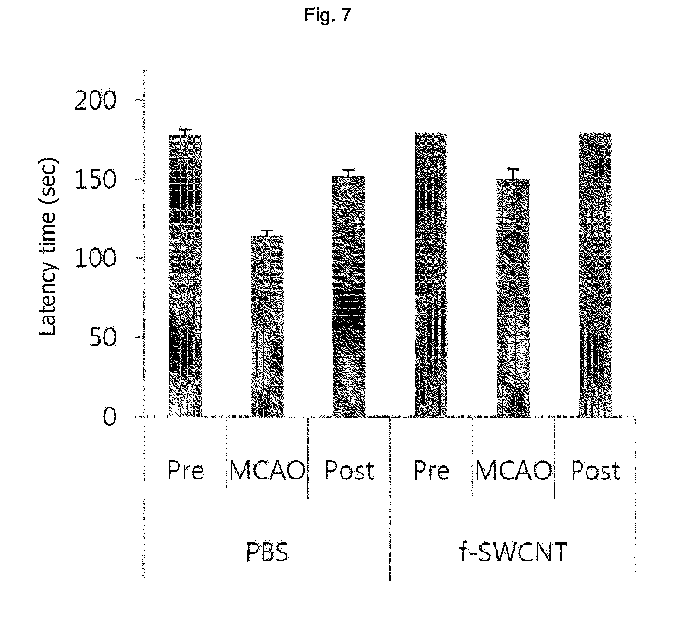

Fig. 7 represents graph of result that the present composition containing the carbon nanotube as an active ingredient is administrated to the stroke model induced by transient middle cerebral artery occlusion (MCAO) and observed to verify inhibitory for brain function damage using Rotarod test. (PBS: PBS-treated group (control), f-SWCNT: the present composition-treated group, Pre: result of Rotarod test before MCAO induction, Post: result of Rotarod test on 7 days after MCAO induction, Y axis: Latency time (unit: sec)).

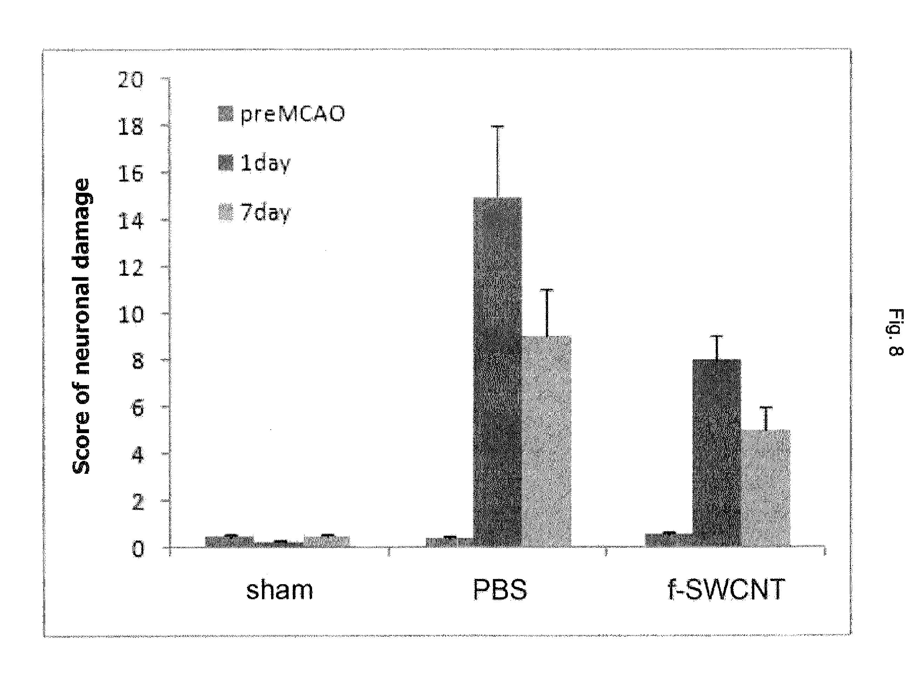

Fig. 8 represents graph of result that the present composition containing the carbon nanotube as an active ingredient is administrated to the stroke model induced by transient middle cerebral artery occlusion (MCAO) and observed to verify inhibitory for brain function damage using Limb placing test. (preMCAO: result of Limb placing test before MCAO induction, 1 day: result of Limb placing test on 1 days after MCAO induction, 7 day: result of Limb placing test on 7 days after MCAO induction, PBS: PBS-treated group (control), f-SWCNT: the present composition-treated group, sham: MCAO-non operated group, Y axis: Score of neuronal damage).

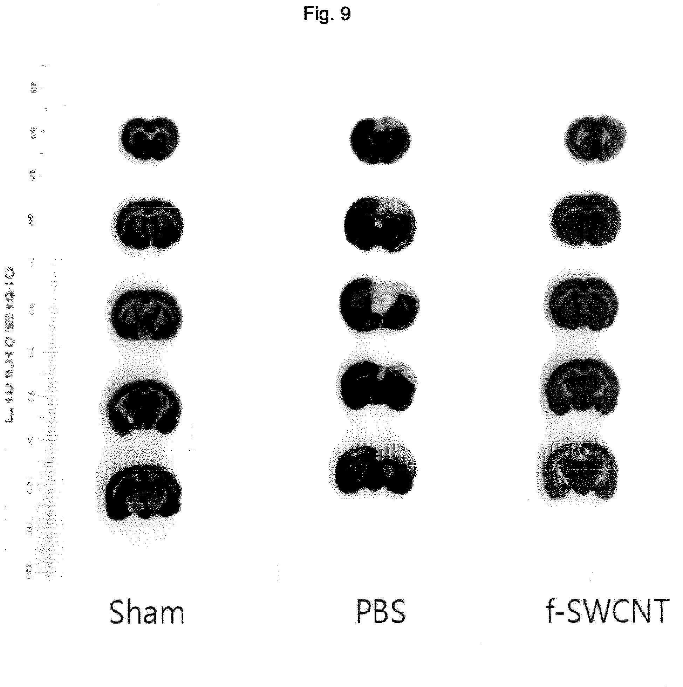

Fig. 9 represents images of result that the present composition containing the carbon nanotube as an active ingredient is administrated to the stroke model induced by transient middle cerebral artery occlusion (MCAO) and observed to verify inhibitory (protection) for cerebral infarction using TTC (Triphenyl tetrazolium chloride) staining. (PBS: PBS-treated group (control), f-SWCNT: the present composition-treated group, sham: MCAO-non operated group).

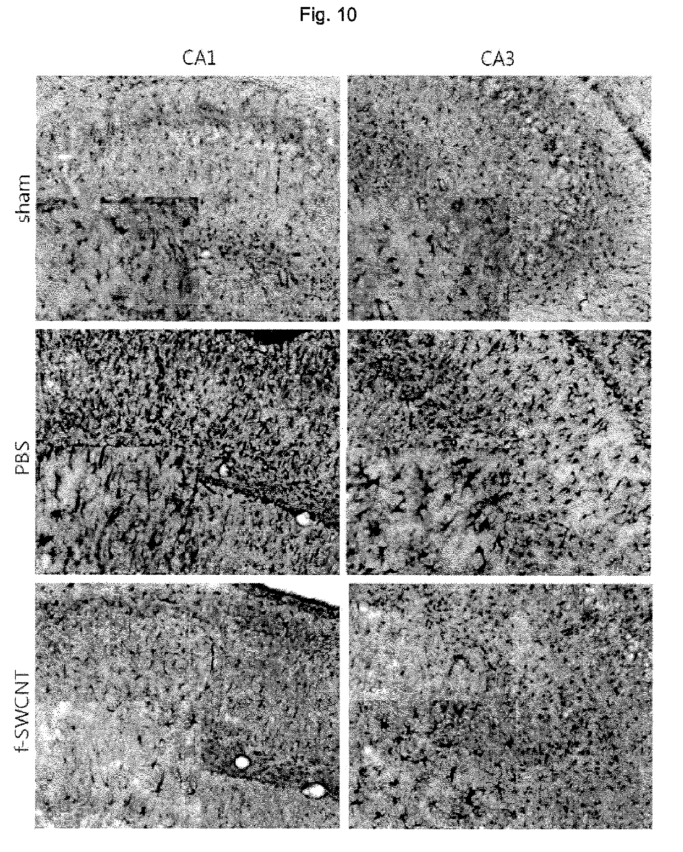

Fig. 10 represents images of result that the present composition containing the carbon nanotube as an active ingredient is administrated to the stroke model induced by transient middle cerebral artery occlusion (MCAO) observed to verify effect for inflammatory responses using immunohistochemistry determining expression of GFAP (glial fibrillary acidic protein) which is a marker for glial activation (PBS: PBS-treated group (control), f-SWCNT: the present composition-treated group, sham: MCAO-non operated group, CA1: cornu ammonis area 1 in hippocampus, CA3: cornu ammonis area 3 in hippocampus).

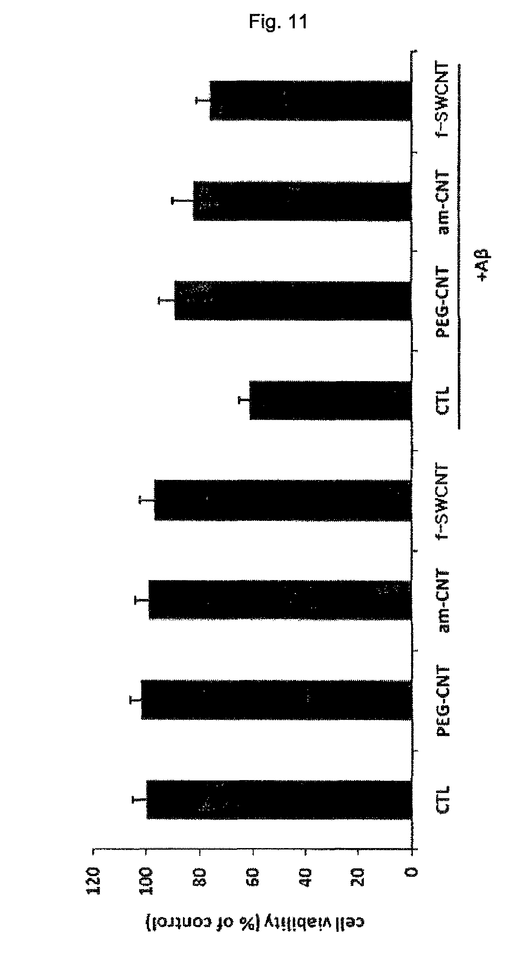

Fig. 11 represents graph of result that the present composition containing the carbon nanotube as an active ingredient is administrated to the Alzheimer's disease model induced by amyloid beta peptide and observed to verify inhibitory (protection) for neurotoxicity using measuring cell viability (CTL: negative control group, f-SWCNT: cabon nanotube functionalized with carboxyl group-treated group, PEG-CNT: cabon nanotube functionalized with polyethylene glycol group-treated group, am-CNT: cabon nanotube functionalized with amide group-treated group, +Aβ: amyloid beta-treated group, Y axis: Cell viability (% of control group).

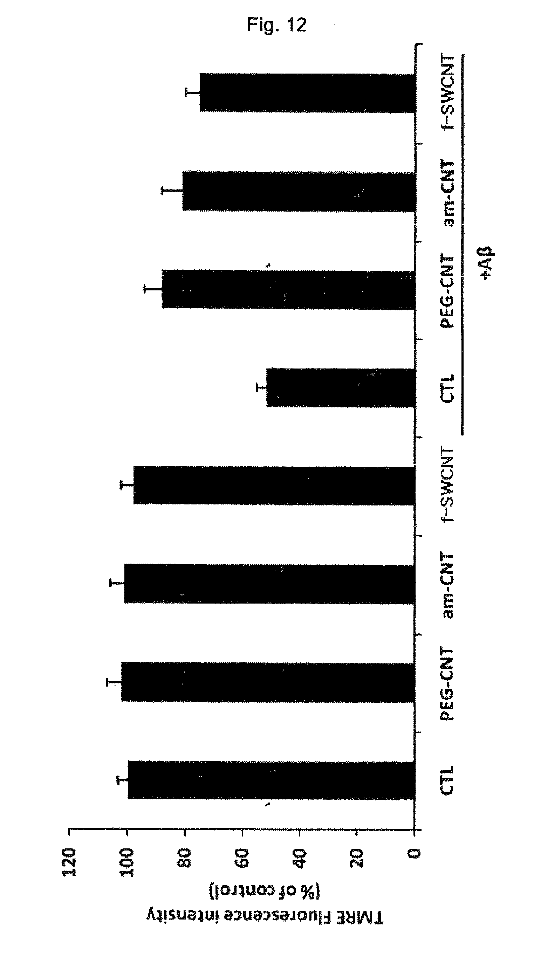

Fig. 12 represents graph of result that the present composition containing the carbon nanotube as an active ingredient is administrated to the Alzheimer's disease model induced by amyloid beta peptide and observed to verify inhibitory (protection) for neurotoxicity using measuring mitochondrial membrane potential (CTL: negative control group, f-SWCNT: cabon nanotube functionalized with carboxyl group-treated group, PEG-CNT: cabon nanotube functionalized with polyethylene glycol group-treated group, am-CNT: cabon nanotube functionalized with amide group-treated group, +Aβ: amyloid beta-treated group, Y axis: TMRE Fluorescene intensity (% of control group).

### Best Mode for Carrying Out the Invention

The present invention will now be described in further detail by examples. It would be obvious to those skilled in the art that these examples are intended to be more concretely illustrative and the scope of the present invention as set forth in the appended claims is not limited to or by the examples.

### <Example 1>

### Preparation of composition containing carbon nanotubes

Single-walled carbon nanotubes used in the present invention were purchased from Sigma aldrich (USA, Cat No. 652490). For each experiment, 0.1 to 1 wt% of carbon nanotubes were dissolved in distilled water and sonicated for 24 hours to increase degree of dispersion before use.

### <Example 2> Effects tests for traumatic brain injury

### <2-1> Preparation of traumatic brain injury animal model

40-45 g weight of ICR male mice (12-week old) were used for animal model having traumatic brain injury. The mice were housed in 21 ± 1°C temperature- and 50 ± 10% humidity-controlled environment under 12 hrs light/dark cycle with free access to food and water, and they were supplied from Korean BioLink Co. (Chungbuk, Korea).

The mouse anesthetized with 8-10 mg/kg of ketamine was placed in Small Animal stereotaxic instrument (David Kopf instrument, Tujunga, USA). Its midline longitudinal scalp was incised with 1 cm long to expose bregma and lambda of skull. A 0.2 cm hole was drilled through the skull with a driller at the point of anterior/posterior -1.82 mm and left/right -2.0 mm from bregma. 2.5mm depth in hippocampus was pressed for 5 min with persistent impact to occur injury using a 17G needle which was made to flat at the end. 20 µg (10 mg/ml, 2 µl) of carbon nanotubes or 2 µl of PBS were injected into the damaged area of the mouse. The incision was sutured and mouse was stabilized. Sham group was anesthetized, incised and sutured without brain injury.

### <2-2> Behavior experiment-Passive avoidance test

To analyze the acquiring and maintenance of learning and memory of the mouse having traumatic brain injury in Example 2-1, passive avoidance test was performed on 28th days after traumatic brain injury as follows: the mouse was trained to move from noisy and light chamber to quiet and dark chamber, and the door was closed. This training was repeated for 5 days once a day at the same time. On 5th days, when the mouse moved to the dark chamber, 0.9 mA of current was provided for 2 sec through mouse foot shock to recognize stimuli. On 6th days, when the mouse in which recognizes the shock entered to the noisy and light chamber, the mouse did not move to the dark chamber. However, when the mouse in which lacks cognitive capability enters to the noisy and light chamber, the mouse moved to the dark chamber. At this moment, the period of latency time in noisy and light chamber was measured to analyze cognitive capability. The latency time was limited to 600 sec.

As a result, as shown in Fig. 1, it was determined that while most of the group injected with PBS did not recognize the shock, most of the group injected with the carbon nanotubes of the present invention effectively recovered learning memory ability.

### <2-3> Histological observation

After animal behavior experiment, the mouse was sacrificed, surgically excised brain tissue. The tissue of each experimental group was fixed in 4% paraformaldehyde for 24 hrs and then dehydrated for 4 days using 15%, 20%, 25% and 30% sucrose solutions. The brain tissues were sliced by a freezing microtome with 20 µm of thickness. The slides of brain tissue sections were stained with H&E and Nissle. Their images were taken using LEICA microscope and digital camera.

As a result, as shown in Figs. 2 and 3, it was determined that while traumatic brain injury in the mouse injected with PBS was progressed to make hole in brain tissue, traumatic brain injury in the mouse injected with the carbon nanotubes of the present invention was regenerated to heal damaged area in brain tissue.

### <Example 3> Effects tests for Parkinson's disease

### <3-1> Measurement test of mitochondrial membrane potential

To evaluate efficacy of the present composition for Parkinson's disease, the carbon nanotubes composition was treated to neuroblast cell line SH-SY5Y with 0.001 wt% of concentration for 1 hr, measured a mitochondrial membrane potential (ΔΨm) and treated with 50 µM of 6-hydroxydopamine to induce neuronal cell damage. After 6 hrs, the mitochondrial membrane potential was measured again. In addition, non-treated carbon nanotubes composition as a control group was treated to the same cell line and measured the mitochondrial membrane potential in the same manner as mentioned above.

Using a fluorescent staining with TMRE (tetramethyl rhodamine ethyl ester, Molecular Probe, Invitrogen, USA), the disruption of mitochondrial membrane potential was measured. The positively charged TMRE is penetrated into mitochondrial membrane with help of mitochondrial membrane potential, and the fluorescene intensity indicates the maintenance of mitochondrial membrane potential. To measure mitochondrial membrane potential each cell line was treated with 100 nM of TMRE and incubated for 15 min in 37°C incubator. The fluorescene intensity was measured using a fluorometer (TECAN, GENios, Swiss) at excitation wavelength of 549 nm and emission wavelength of 574 nm, and membrane potential was expressed with relative value to the intensity.

As a result, as shown in Fig. 4, it was determined that while the mitochondrial membrane potential at 6hrs after 6-hydroxydopamine treatment in control group decreased remarkably by 40 % of normal level, the mitochondrial membrane potential in the carbon nanotubes of the present invention maintained with no more less 60 %. It could be understood that the carbon nanotubes also may inhibit dopaminergic neuronal cell damage by 6-hydroxydopamine *in vitro.*

### <3-2> Preparation of Parkinson's disease animal model

40-45 g weight of ICR male mice were used for animal model having Parkinson's disease. The mice were housed in 21 ± 1°C temperature- and 50 ± 10% humidity-controlled environment under 12 hrs light/dark cycle with free access to food and water, and they were supplied from Samtako (Gyeonggi-do, Korea).

The mice were divided into three groups as follows: sham; PBS-pretreated group; and carbon nanotubes-pretreated group. The mouse anesthetized with 70 mg/kg of ketamine and 15 mg/kg of xylazine was placed in Small Animal stereotaxic instrument. 20 µg (10 mg/ml, 2 µl) of carbon nanotubes or 2 µl of PBS (0.1M Phosphate buffered saline, pH7.4) were injected into the desired region (anterior/posterior 0.22 mm; left/right 1.1 mm from bregma; and depth 2.2 mm) using Hamilton syringe at flow rate of 0.5 µl/min. Sham group was anesthetized, incised and sutured without injection. After 3 days, 10 µl of 6-hydroxydopamine (60HDA)(4 mg/ml with 0.2 mg/ml L-ascorbic acid) was injected into right striatum (anterior/posterior 0.8 mm; left/right 2.0 mm from bregma; and depth 3.3 mm).

### <3-3> Behavior experiment-Rotation test

2 weeks after 6-hydroxydopamine injection, 0.5 mg/kg of apomorphine was administrated subcutaneously to determine induction of Parkinson's disease model and protection efficacy. Asymmetric rotation caused by apomorphine was measured using rotometer and recorded for 45 min. Rotation numbers were measured using automated rotation measuring instrument which is prepared by the present inventors using modifying Ungerstedt (Ungerstedt, U. 1971, Adipsia and aphagia after 6-hydroxydopamine induced degeneration of the nigro-striatal dopamine system. Acta Physiol Scand 82, (suppl 367) 69-92).

As a result, as shown in Fig. 5, it was determined that while the group injected with PBS rotated to one-direction in which is a representative symptom of Parkinson's disease and the rotation numbers during 45 min were nearly 180 times, the rotation numbers during 45 min in the group injected with the carbon nanotubes of the present invention were only a little over 50 times. Therefore, it could be understood that the composition of the present invention inhibits to occur Parkinson's disease.

### <3-4> Histological observation

The slides of brain tissue sections were prepared in the same manner as that for the method of Example 2-3. To increase permeability of tissue, the slides were washed with PBST (PBS in 0.1% triton x-100) and incubated in 3 % H₂O₂ for 5 min. All washing solutions were used as PBST. The slides were blocked with 3 % Bovine serum albumin for 1 hr, followed by incubation with TH antibody which was diluted to 1:100 (anti-Tyrosine hydroxylase polyclonal antibody, sc-7847, Santacruz, USA) at 4°C overnight. The slides were washed with PBST, incubated with anti-goat biotinylated antibody and ABC solution (PK-6105, VECTASTAIN, USA) for 1 hr at 37°C, respectively, and colorized with DAB (D-5637, Sigma, USA).

As a result, as shown in Fig. 6, TH-positive neuronal cells in the area injected with 6-hydroxydopamine of the group injected with PBS were decreased, demonstrating that death of dopaminergic neuronal cells was induced. In contrast, the group injected with the carbon nanotubes of the present invention was non-observed to show stained dopaminergic neurons. Therefore, it was determined that the composition of the present invention inhibits Parkinson's disease.

### <Example 4> Effects tests for stroke

### <4-1> Preparation of stroke animal model

An ischemic stroke model was prepared by transient middle cerebral artery occlusion (MCAO) to verify effects of the carbon nanotubes for protecting and treating brain.

220-270 g weight of male wistar rats were used for animal model. The rats were housed under 12 hrs light/dark cycle with free access to food and water. The rats were divided into three groups as follows: sham; PBS group; and carbon nanotubes group. The rat anesthetized with 80 mg/kg of ketamine and 40 mg/kg of rumpun was placed in Small Animal stereotaxic instrument. 0.04 µg (1 wt%, 4 µl) of carbon nanotubes or 4 µl of PBS were injected into cerebral lateral ventricle (anterior/posterior 0.8 mm; left/right 1.4 mm from bregma; and depth 3.6 mm). After 7 days, the rats were anesthetized again. A middle incision was made in the neck, and right common carotid artery was carefully exposed and dissected from

the surrounding tissue. External and common carotid arteries were occlusived by 5-0 silk suture. Internal carotid artery was carefully dissected from the surrounding tissue and occlusived loosely by 5-0 silk suture. A 5-0 nylon suture (Ethicon, Johnson & Johnson, Somerville, NJ, USA)) was inserted at the bifurcation of the external and internal carotid arteries. 90 min later, reperfusion was performed. In the sham group, the middle cerebral artery was not occluded.

### <4-2> Behavior experiment-Rotarod test

1 day after induction of MCAO, Rotarod test was subjected to evaluate brain function damage by behavioral changes. The Rotarod test is a performance test that the rats try to stay on the rotating drum without falling to the ground and the length of time that a given animal stays on this rotating rod is measured. The rotational speed accelerated from 6 to 19 rpm over 3 min. The rats used in this experiment were trained to remain on a rotating drum for 3 days before induction of stroke. The time at which the rat ran on the drum (latency time) was measured repeatedly three times to obtain the average value.

As a result, as shown in Fig. 7, while all rats could stay on rotating drum until 180 sec before MCAO incuction, latency time of the group injected with PBS was remarkably decreased in Rotarod test on 1 day after MCAO induction. 7 days after MCAO induction, latency time of that was 150 sec, demonstrating that it was not recovered remarkably. In contrast, latency time of the group injected with the carbon nanotubes of the present invention represented more than 150 sec on 1 day after MCAO induction. In addition, 7 days after MCAO induction, latency time of that was nearly normal state. Therefore, it could be understood that brain damage by MCAO and behavioral changes due to the brain damage were recovered.

### <4-3> Behavior experiment-Limb placing test

Limb placing test is a performance test evaluating sensomobility of forelimb and hindlimb responding to stimuli for senses of vision and position.

First, forward limb placing test was executed in the manner that an examiner grasped rat tail and lifted it into the air, and the extension of forelimbs was evaluated and scored as follows: normal extension in the forelimbs = 0 points and abnormal flexion in the right forelimb = 3 point. Second, lateral limb placing test was executed in the manner that an examiner grasped rat trunk with free access to movements of forelimbs and pulling down horizontally onto the table. At this time, where the rat tries to grip the table with stretching its forelimbs, the behavior is determined to normal response. The stretch of the forelimbs was evaluated sequentially three times and scored as follows: 3 times of normal response in the forelimbs = 0 point, 1 time of abnormal response in the forelimbs = 1 point, 2 time of abnormal response in the forelimbs = 2 point and 3 time of abnormal response in the forelimbs = 3 point.

As a result, as shown in Fig. 8, while the score of sensomobility which was measured before induction of stroke was nearly 0 point with normal state, the score of sensomobility which was measured after induction of stroke in the group injected with PBS was remarkably increased by more than 15 point, demonstrating that sensomobility was damaged seriously. In contrast, it was determined that the group injected with single-walled carbon nanotubes was inhibited the damage. In addition, 7 days after induction of stroke, sensomobility was still maintained. Therefore, it could be understood that carbon nanotubes may effectively inhibit neurological damage.

### <4-4> Verification of stroke and histological observation

24 hrs after induction of middle cerebral artery occlusion, the rat brains were harvested and slides of brain tissue sections were prepared in the same manner as that for the method of Example 2-3. The slides were stained with TTC (Triphenyl tetrazolium chloride) to verify stroke. In normal cells, TTC reacts with mitochondrial dehydrogenase to result red color. However, where dehydrogenase was lost, cells were represented white color since they were non-stained. Such results indicate that white area demonstrates range of stroke and the presence or absence of irreversible damage of cells.

As a result, as shown in Fig. 9, while infarcted regions were observed in cerebral cortex of the group injected with PBS, there were no infarcted regions in the group injected with the carbon nanotubes at 7 days before induction of MCAO.

In addition, to verify the effect of carbon nanotubes for the MCAO-induced inflammatory responses, immunohistochemistry was carried out using GFAP (glial fibrillary acidic protein) which is a marker for glial activation.

To increase permeability of tissue, the slides were washed with PBST (PBS in 0.1% triton x-100) and incubated in 3 % H₂O₂ for 5 min. All washing solutions were used as PBST. The slides were blocked with 3 % Bovine serum albumin for 1 hr, followed by incubation with GFAP antibody which was diluted to 1:100 (anti-GFAP rabbit polyclonal antibody, ab-7260, abcam, UK) at 4°C overnight. The slides were washed with PBST, incubated with anti-rabbit biotinylated antibody and ABC solution (PK-6101, VECTASTAIN, USA) for 1 hr at 37°C, respectively, and colorized with DAB (D-5637, Sigma, USA). GFAP expressions of CA1 (cornu ammonis area 1) and CA3 (cornu ammonis area 3) in Hippocampus which is responsible for memory and learning ability were observed. As a result, as shown in Fig. 10, it was determined that the star-shaped size and the number of astrocytes in the group injected with PBS were increased and activated. Such results indicate that inflammatory responses were increased. In contrast, it was determined that glial activation level in the group injected with the carbon nanotubes at 7 days before induction of MCAO was maintained in the same level of sham group. Such result indicates that the present carbon nanotubes enable the nerve cells and brain tissue to endure against MCAO, and protect brain cells.

### <Example 5> Effects tests for Alzheimer's disease

The present carbon nanotubes neuroprotective effects on neurotoxicity of amyloid beta peptide were verified. The amyloid beta peptide is a cause substance and a marker of Alzheimer's disease.

### <5-1> Measurement test of neuronal cell viability

Human neuroblast cell line SH-SY5Y was treated with 10 µM of amyloid beta protein. After 36 hrs, the neuronal cells were measured their viabilities.

SH-SY5Y cultured in plate was incubated with 0.001 wt% of the carbon nanotubes composition at 37°C in 5% CO₂ incubator. Following the incubation of 1 hr, 10 µM of amyloid beta protein was treated to SH-SY5Y. After 36 hrs, the cells were measured their viabilities as follows: 10 µM of alarmablue solution (Serotec, UK) was added to each well and incubated for additional 3-hr. Reduced alarmablue were measured at 570 nm using ELISA reader (Molecular Devices, Sunnyvale, CA) for analyzing the activity of mitochondria. The absorbance background was obtained at 600 nm. The cell viability was calculated with the following formula: Cell viability = [(sample count) - (blank count) / (non-treated control count)- (blank count)] x 100.

As a result, as shown in Fig. 11, it was determined that while the cell death in the group non-treated carbon nanotubes after treatment of amyloid beta was represented by approximately 50 %, the cell viability in the group injected with the carbon nanotubes before 1 hr was increased by approximately 70%.

### <5-2> Measurement test of mitochondrial membrane potential

The cells treated in the same manner as that for the method of Example 5-1 were measured their mitochondrial membrane potential. The mitochondrial membrane potential was measured using TMRE (Molecular Probes, Invitrogen, USA) in the same manner as that for the method of Example 3-1.

As a result, as shown in Fig. 12, it was determined that the present carbon nanotubes may inhibit damage of mitochondrial membrane potential caused by amyloid beta and protect function of mitochondrial membrane potential. In addition, it could be understood that the present carbon nanotubes may provide protective effects in cellular level as well as tissue level, whereby the cell viabilities are increased through protection of mitochondrial membrane potential.

Having described a preferred embodiment of the present invention, it is to be understood that variants and modifications thereof falling within the spirit of the invention may become apparent to those skilled in this art, and the scope of this invention is to be determined by appended claims and their equivalents.

### Industrial applicability

The present invention provides a composition for preventing or treating brain disease, a composition for treating traumatic central nervous system injuries or a composition for protecting cranial nerves, containing carbon nanotubes as an active ingredient. The composition of the present invention enables patients to recover from physical damage to the brain, exhibits superior efficacy for inhibiting the onset of Parkinson's disease and strokes in animal models for Parkinson's disease and strokes, and the cytotoxic effects of beta amyloid in beta amyloid toxicity tests. Therefore, the composition of the present invention can be effectively used in the preparation of medicine for protecting cranial nerves, therapeutic agents for preventing or treating brain disease, or therapeutic agents for treating traumatic central nervous system injuries, whereby the present invention is industrially applicable.

## Claims

1. A composition for preventing or treating a brain disease, comprising a carbon nanotube as an active ingredient.

2. The composition according to claim 1, wherein the brain disease is a degenerative brain disease or an ischemic brain disease.

3. The composition according to claim 2, wherein the degenerative brain disease is selected from the group consisting of Alzheimer's disease, Parkinson's disease, dementia, progressive supranuclear palsy, multiple system strophy, Olivopontocerebellar atrophy (OPCA), Shy-Drager syndrome, Striatonigral degeneration, Huntington's disease, amyotrophic lateral sclerosis (ALS), essential tremor), cortico-basal ganlionic degeneration, Diffuse Lewy body disease, Parkinson-ALS-dementia complex of Guam and Pick's disease.

4. The composition according to claim 2, wherein the ischemic brain disease is selected from the group consisting of thrombosis, embolism, transient ischemic attack, lacune, stroke, cerebral hemorrhage, cerebral infarction, head trauma, cerebral circulation disorder and coma.

5. The composition according to claim 1, wherein the carbon nanotube is functionalized.

6. The composition according to claim 5, wherein the carbon nanotube is functionalized with one or more functional groups selected from the group consisting of carboxyl group, thiol group, amide group, poly(ethylene glycol) group, octadecylamine group and polyaminobenzene sulfonic acid group.

7. A composition for treating traumatic central nervous system injuries, comprising a carbon nanotube as an active ingredient.

8. The composition according to claim 7, wherein the traumatic central nervous system injuries are traumatic brain injury (TBI) or traumatic spinal cord injury (TSCI).

9. The composition according to claim 7, wherein the carbon nanotube is functionalized.

10. The composition according to claim 9, wherein the carbon nanotube is functionalized with one or more functional groups selected from the group consisting of carboxyl group, thiol group, amide group, poly(ethylene glycol) group, octadecylamine group and polyaminobenzene sulfonic acid group.

11. A composition for protecting cranial nerves, comprising a carbon nanotube as an active ingredient.

12. The composition according to claim 11, wherein the carbon nanotube is functionalized.

13. The composition according to claim 12, wherein the carbon nanotube is functionalized with one or more functional groups selected from the group consisting of carboxyl group, thiol group, amide group, poly(ethylene glycol) group, octadecylamine group and polyaminobenzene sulfonic acid group.

14. A use of a carbon nanotube for manufacturing a therapeutic agent for preventing or treating a brain disease.

15. A use of a carbon nanotube for manufacturing a therapeutic agent for preventing or treating traumatic central nervous system injuries.

16. A use of a carbon nanotube for manufacturing a medicament for protecting cranial nerves.

17. A method for preventing or treating a brain disease, comprising administering to a subject in need thereof a therapeutically effective amount of a carbon nanotube.

18. A method for preventing or treating a traumatic central nervous system injury, comprising administering to a subject in need thereof a therapeutically effective amount of a carbon nanotube.

19. A method for protecting cranial nerves, comprising administering to a subject in need thereof a therapeutically effective amount of a carbon nanotube.
